# EUROPEAN PATENT APPLICATION

(11) **EP 1 216 716 A1**
(43) Date of publication of application: **26.06.2002**
(21) Application number: 00127963.7
(22) Date of filing: 20.12.2000
(51) Int. Cl.: A61L 9/12

(54) **Ceramic sculpture for storing and releasing fragrance and method of making the same**

(71) Applicant: Chang, Wen-Cheng, Taipei-Taiwan (CN)
(72) Inventor: Chang, Wen-Cheng, Taipei-Taiwan (CN)
(74) Representative: Serwe, Karl-Heinz, Dipl.-Ing.

(57) **Abstract**

A ceramic sculpture for storing and releasing fragrance is provided which includes a bowl-like body ofthe ceramic sculpture with an indentation at the bottom with a vapor permeation barrier on the surface of the indentation; a unglazed ceramic base embedded into the indentation of the bowl-like body of the ceramic sculpture; and a fragrant substance placed in the indentation and the base, while fragrance is absorbed and released from the surface of the base. A method of making the ceramic sculpture for storing and releasing fragrance is also provided.

## Description

### BACKGROUND OF THE INVENTION

### 1. Field of invention

The present invention relates to a ceramic sculpture for storing and releasing fragrance, and the method of making such structures.

### 2. Description of related art

Generally, unglazed ceramics is known to have absorption capabilities and absorb liquid such as water or essential oils. Also, its absorption capability can be improved by changing the composition of ceramic material before it is being burnt in a kiln.

In prior art, fragrance is added to an unglazed ceramic product that has been burnt in a kiln, or alternatively, a container is installed inside the ceramic product for holding fragrant substances in order to make the ceramic product aromatic. However such ceramic products are not glazed to allow discharge of its fragrance. As such, the uses for these ceramic products are limited and they cannot be used as common household ceramic products.

To avoid the above-mentioned technical problem, the inventors of the present invention improved the capability of the ceramic product to store and release fragrance without changing the original formula for raw materials and the shape and functions of the household ceramic product. In addition, the present invention can effectively control the amount of fragrance released from the ceramic sculpture.

### SUMMARY OF THE INVENTION

The primary objective of the present invention is to provide a ceramic sculpture for storing and releasing fragrance, which allows a common ceramic product to easily maintain and release fragrance for a longer period of time. Another objective of the present invention is to provide a method of making a ceramic sculpture for storing and releasing fragrance, which is simple in production procedure and low in production cost when compared with the prior art. The present invention relates to a ceramic sculpture for storing and releasing fragrance, which includes a bowl-like body of the ceramic sculpture with an indentation at the bottom with a vapor permeation barrier on the surface of the indentation; a unglazed ceramic base embedded into the indentation of the bowl-like body of the ceramic sculpture; and a fragrant substance placed in the indentation and the base while fragrance is absorbed and released from the surface of the base.

In the above-described structure, a space is formed between the surface of the indentation at the bottom of the ceramic sculpture and the base embedded into the indentation, wherein a piece of refractory wool is inserted. The base is formed with holes for the fragrance to be injected into the said space under high pressure. As such, the fragrance can be stored and discharged because of the space formed and the base, as well as the absorption capability of the ceramic base and/or the refractory wool itself.

The method of making the ceramic sculpture for storing and releasing fragrance under the present invention includes the following steps:
prepare the main body of the unglazed ceramic sculpture and the unglazed base separately;
glaze the surface of the indentation. The layer of glaze acts as a barrier to prevent the fragrance from seeping upwards and minimize wastage;
insert the base into the indentation of the ceramic sculpture and seal with porcelain clay;
burn the unglazed ceramic body in a kiln at a temperature of at least 1050°C; and
after the burnt ceramic sculpture has cooled down, inject the fragrant substance into the base under a high pressure, so that the space between the surface of the indentation and the base is completely filled with the fragrant substance.

In the method described above, the remaining unglazed portion of the ceramic sculpture is glazed after the base is inserted into the indentation of the ceramic sculpture and sealed with porcelain clay. On the other hand, while the base and the indentation are being embedded together, it is also possible to leave a small space in the glazed indention, wherein a piece of refractory wool (which has a heat-resistance level of over 1300 °C, and its material is similar to sponge) is inserted. Both the refractory wool and the base have the function of absorbing, retaining, and discharging the fragrance. The preferred temperature for the above-mentioned burning process is 1080°C to 1280°C. Furthermore, the fragrant substance to be used in the above-described method are liquid substance such as essential oils.

### BRIEF DESCRIPTION OF DRAWINGS

The invention can be more fully understood by reading the following detailed description of the preferred embodiments, with reference made to the accompanying drawings, wherein:
Fig.1 shows the elements which constitute the ceramic sculpture for storing and releasing fragrance.
Fig. 2 shows the structure of the bottom portion of the ceramic sculpture for storing and releasing fragrance in the first preferred embodiment, prior to the injection of the fragrant substance.
Fig. 3 shows the structure of the bottom portion of the ceramic sculpture for storing and releasing fragrance in the first embodiment.
Fig. 4 shows the elements of the ceramic sculpture for storing and releasing fragrance in another style of embodiment.
Fig. 5 shows the structure of the bottom portion of the ceramic sculpture for storing and releasing fragrance in the second embodiment.

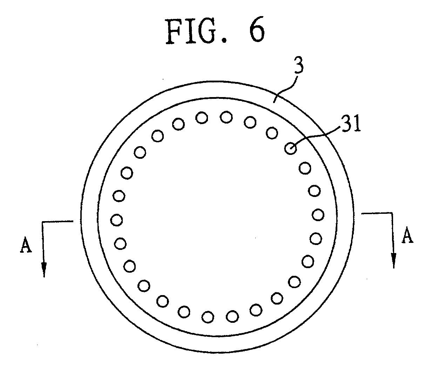

Fig. 6 is a vertical view of the base.
Fig. 7 is a flow chart illustrating the method of making the ceramic sculpture for storing and releasing fragrance.
Fig. 8 shows the water-absorption of the base.
Fig. 9 is a diagram showing the relationship between the height of water absorption by the base and the duration of immersion.

### DETAILED DESCRIPTION OF PREFERRED EMBODIMENTS

The following preferred embodiments of the ceramic sculpture for storing and releasing fragrance are disclosed to illustrate the content of technology and the scope of claims of the present invention. However, these preferred embodiments are not meant to limit the scope of the present invention.

### FIRST PREFERRED EMBODIMENT

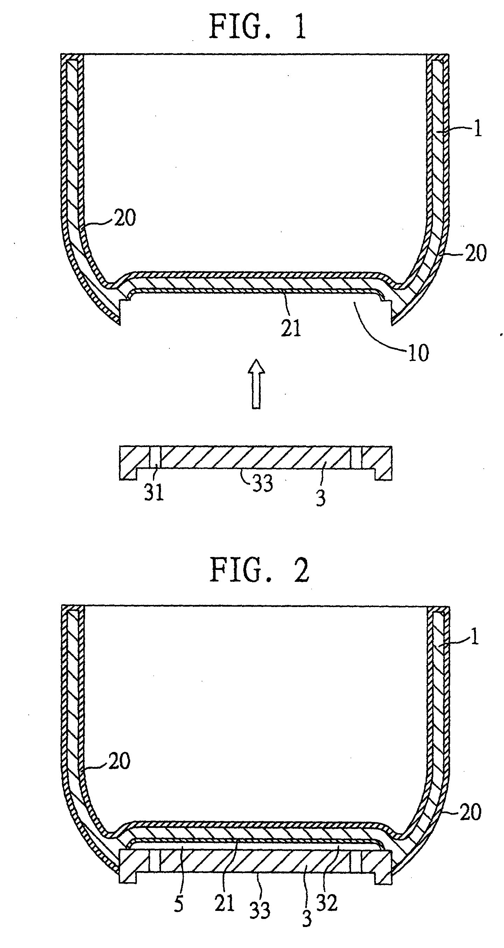

As shown in Figures 1 to 3, the ceramic sculpture for storing and releasing fragrance is constituted by a Bowl-like Ceramic Body 1, at the bottom of which Indentation 10 is formed. Base 3 is inserted into Indentation 10. Space 32 is formed between Indentation 10 and Base 3. A layer of Glaze 20 is formed on the outer surface of the Bowl-like Ceramic Body 1, allowing Bowl-like Ceramic Body 1 to be used as a container or other utensils. Furthermore, Indentation 10 is coated with Glaze Layer 21, which acts as a barrier, preventing the fragrance (as described below) from seeping towards the outer-wall of the Bowl-like Ceramic Body.

Base 3 is an unglazed piece of ceramic that underwent the burning process at a high temperature and is capable of absorbing and storing fragrant substances or liquid fragrance. A number of Small Holes 31 are formed along the edges of Base 3. The purpose of these Small Holes 31 is to allow the release of vapor created by Glaze Layer 21 and the vapor that exists between Indentation 10 and Base 3, when Base 3 is embedded into Indentation 10 and then burnt inside a kiln. On the other hand, fragrant substances can be injected with a high pressure injector through these Small Holes 31 after the burning process at high temperatures, so that the fragrant substance is placed into Base 3 and Space 32, and the fragrance is released from the small holes or the exposed surface of Base 3.

The fragrant substance can be any volatile liquid in the form of synthetic or natural products, such as aromatic essential oil, hinoki oil, sandalwood oil, or cironella oil, and be used in the present invention.

### SECOND PREFERRED EMBODIMENT

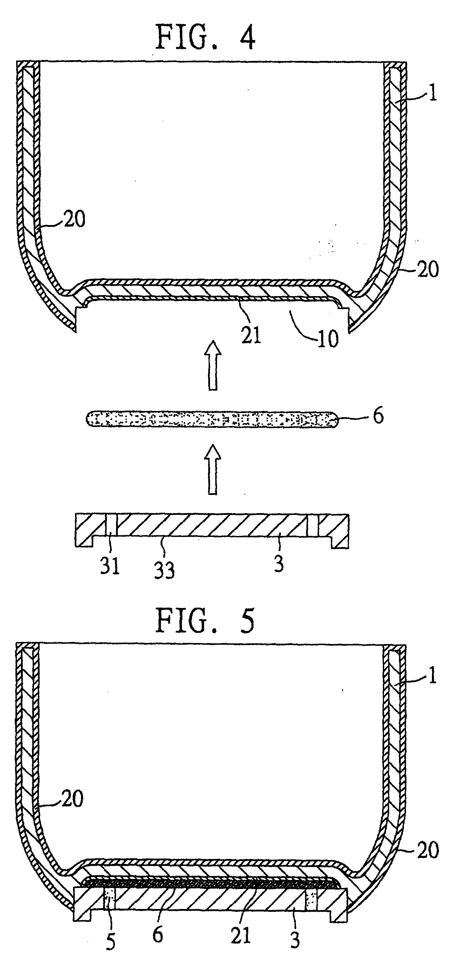

In addition to the preferred embodiment, Figures 4 and 5 show that a piece of Refractory Wool 6 can be inserted in between Indentation 10 and Base 3. The Refractory Wool 6 has a melting point higher than the burning temperature of the Bowl-like Ceramic Body 1 by approximately 50°C to 300°C, so that the ceramic sculpture is not kept too dense during the burning process. Upon the completion of the burning process, the fragrant substance is injected, more fragrance is be absorbed, and a longer fragrance-discharging period is maintained. The Bowl-like Ceramic Body 1 and Base 3 in both the first and the second preferred embodiment are the same individually; thus no repeated explanation is required about them, and the same symbols are used.

### FIRST EMBODIMENT OF MANUFACTURE

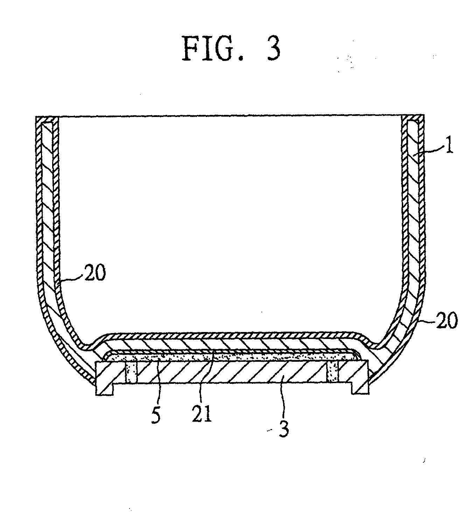

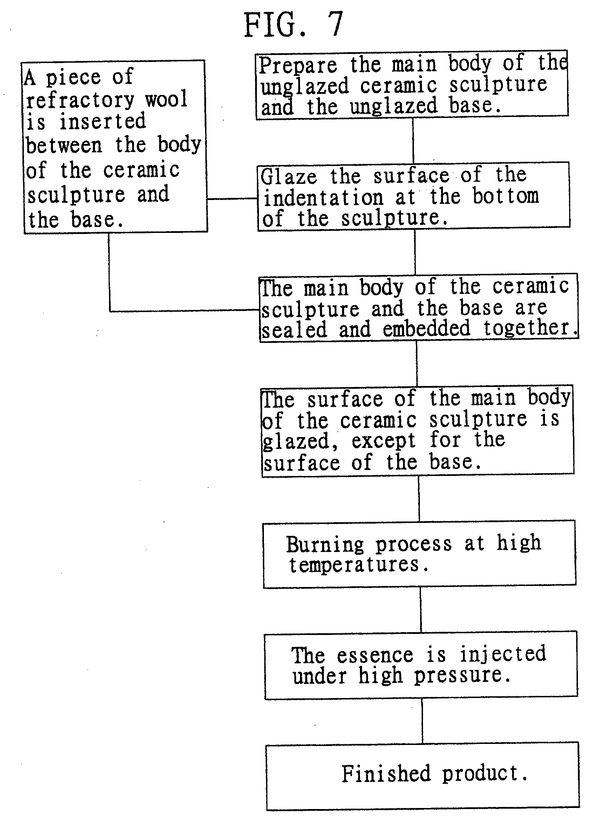

As shown in Fig. 7, the first thing is to prepare the unglazed Bowl-like Ceramic Body 1 and the unglazed Base 3 with Small Holes 31. Then coat the surface of Indentation 10 with a layer of Glaze 21. The layer of glaze serves as a barrier which prevents the fragrance from seeping upwards and causing wastage. Then insert Base 3 into Indentation 10, so that Space 32 is formed between the two, then seal with porcelain clay. The surface of the sealed ceramic sculpture is then coated with a layer of Glaze 20, except for the indentation of Bowl-like Ceramic Body 1 and Surface 33 (which is the bottom surface of Base 3). The sculpture is then heated at a high temperature of 1080 to 1280°C. After the ceramic sculpture has cooled down, a fragrant substance is injected under a high pressure. Essence 5 is injected under the high pressure through Small Holes 31 of Base 3. Space 32 (which is a space formed between the bottom of Bowl-like Ceramic Body 1 and the Base 3) is filled with Essence 5, which is in turn absorbed, stored and released through Base 3. Figure 3 shows the structure of the base which is made to store and discharge fragrance.

In the present invention, small holes are made during the production of the base structure to allow essence to be injected under high pressure and then absorbed and released through the base. Also, since the essence is injected only after the ceramic sculpture has been heated, the appearance and functions of the ceramic product will not be affected.

Also disclosed in the present invention is another structure of the Ceramic Sculpture for Storing & Releasing Fragrance, wherein a piece of refractory wool is inserted into a small space saved in the glazed indentation at the bottom of the ceramic sculpture when the base is embedded onto the bowl-like ceramic body.

### SECOND EMBODIMENT OF MANUFACTURE

As shown in Fig. 7, in a manner similar to the production method of the first preferred embodiment, an unglazed Bowl-like Ceramic Body 1 and an unglazed Base 3 are prepared individually. Then Indentation 10 at the bottom of the unglazed Bowl-like Ceramic Body 1 is coated with a layer of Glaze 21. After Refractory Wool 6 is inserted under the layer of Glaze 21, the subsequent procedures of sealing with porcelain clay, coating with glaze and burning at high temperatures are carried out in the some manner as set forth in the first preferred embodiment. Essence 5 is then injected under a high pressure through Small Holes 31 on Base 3, so that Essence 5 is absorbed by Refractory Wool 6 and Base 3, and the base structure that is made to store and discharge fragrance is thus produced.

In the above embodiment, the purpose of inserting in the refractory wool is to increase the efficiency of absorbing and retaining the essence. Factors to be considered while choosing the material of the refractory wool include the fact that, ceramic products are usually required to undergo a burning process at high temperatures of 1080°C to 1280°C, and since refractory wool is highly heat-resistant, its tendency to absorb like a sponge is not affected.

In the present invention, the thickness of the refractory wool and the ceramic base can be adjusted; or alternatively, the number and size of the small holes in the base can be changed to effectively control the rate at which essence is released.

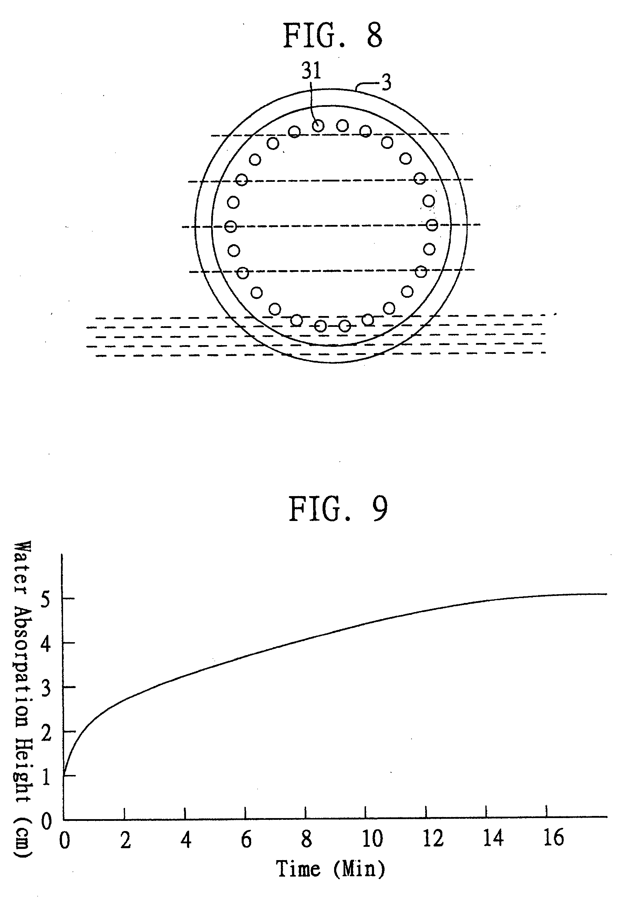

Also in the present invention, the absorption performance of the refractory wool (which is able to absorb, store and release fragrance) and the base are tested and the results are as follows.
(1) Absorption test of the refractory wool: 1.1g of refractory wool can absorbs 6.7g of essence.
(2) Absorption test of the base: As shown in Fig. 8 and Fig. 9, water has replaced essence; a sample Base 3 (net weight: 19G; thickness: 0.4 cm) is vertically placed into the water (1 cm of depth) on its side. The test result, measured by the height of water-absorption by the base immersed in the water after 0.5, 3, 8 and 16 minutes, is shown in the following table.

| Time (min) | Height of Absorption |
|---|---|
| 0 | 1 |
| 0.5 | 2 |
| 3 | 3 |
| 8 | 16 |
| 16 | 5 |

The relationship between the height of water-absorption and time is shown in Fig. 9. Also, after 16 minutes of being soaked in water, the sample Base 3 weighed 23g; in other words, the amount of water absorbed was about 4g.

The quantity absorbed by the ceramic base varies with its size.

During the actual production process when the essence was injected under a high pressure into the base, the test results indicated that liquid substance such as essence can be completely absorbed within 3 seconds.

In conclusion, the ceramic sculpture for storing and releasing fragrance allows a common ceramic product to easily maintain and release fragrance for a longer period of time.

The Sculpture is useful as a household ceramic product, most of which must undergo a burning process whereby the ceramic is securely placed on the kiln cart; therefore the design of the ceramic product must include a base. As such, the present invention is particularly useful for a household ceramic product designed with a base structure, such as common ceramic products, mugs, containers, vases, bathroom ware, decorative ceramics, etc.

On the other hand, the ceramic sculpture for storing and releasing fragrance created under the present invention has many advantages; it is simple to manufacture and the cost of production is low. In addition, the material, appearance and applications of the household ceramic products will not be affected.

The invention has been described using exemplary preferred embodiments. However, it is to be understood that the scope of the invention is not limited to the disclosed embodiments. On the contrary, it is intended to cover various modifications and similar arrangements. The scope of the claims, therefore, should be accorded the broadest interpretation so as to encompass all such modifications and similar arrangements.

## Claims

1. A ceramic sculpture for storing and releasing fragrance, comprising:
a bowl-like body with a indentation at the bottom thereof, the bowl-like body being formed with a layer of vapor permeation barrier on the surface of the indentation;
an unglazed ceramic base disposed in the indentation; and
a fragrant substance that is absorbed and retained by the base while fragrance is released from the surface of the base.

2. The ceramic sculpture as set forth in claim 1, wherein a space is formed between the surface of the said indentation and the base embedded into this indentation.

3. The ceramic sculpture as set forth in claim 2, wherein a refractory wool is inserted into the said space.

4. The ceramic sculpture as set forth in claim 1, wherein the base is formed with small holes to allow injection of fragrant substances into the base and/or the refractory wool.

5. The ceramic sculpture as set forth in claim 1, wherein the vapor permeation barrier formed on the surface of the indentation is a layer of glaze.

6. The ceramic sculpture as set forth in claims 1, wherein the fragrant substance is an essence.

7. The method of making the ceramic sculpture for storing and releasing fragrance as set forth in claim 1, comprising the steps of:
preparing the main body of the unglazed ceramic sculpture and the unglazed base separately;
glazing the surface of the indentation.
inserting the base into the indentation of the ceramic sculpture and seal with porcelain clay;
burning the unglazed ceramic body at a temperature of the least 1050°C;
injecting the fragrant substance into the base under a high pressure after the burnt sculpture has cooled down, so that the base is completely filled with the fragrant substance.

8. The method as set forth in claim 7, wherein the remaining portion of the ceramic sculpture other than the indentation is to be coated with glaze upon completion of inserting the base into the indentation of the ceramic sculpture and seal with porcelain clay.

9. The method as set forth in claim 7, wherein the base is formed with small holes through which the essence is injected.

10. The method as set forth in claim 7, wherein the refractory wool is inserted when the base is embedded into the indentation.

11. The method as set forth in claim 7, wherein both the base and the refractory wool are injected with essence.
